# EUROPÄISCHE PATENTSCHRIFT

(11) **EP 2 713 963 B1**
(45) Veröffentlichungstag und Bekanntmachung des Hinweises auf die Patenterteilung: **26.07.2017**
(21) Anmeldenummer: 12723707.1
(22) Anmeldetag: 29.05.2012
(51) Int. Cl.: A61F 2/90, D04C 1/08

(54) **MEDIZINISCHE VORRICHTUNG ZUR EINFUHR IN EIN HOHLORGAN**
MEDICAL DEVICE FOR INTRODUCTION INTO A HOLLOW ORGAN
DISPOSITIF MÉDICAL DESTINÉ À ÊTRE INTRODUIT DANS UN ORGANE CREUX

(30) Priorität: 31.05.2011 DE 102011102935
(43) Veröffentlichungstag der Anmeldung: 09.04.2014
(73) Patentinhaber: Acandis GmbH & Co. KG, 76327 Pfinztal (DE)
(72) Erfinder: BAIDINGER, Otto, 75175 Pforzheim (DE)
(74) Vertreter: Kilchert, Jochen
(86) Internationale Anmeldenummer: PCT/EP2012/059951
(87) Internationale Veröffentlichungsnummer: WO 2012/163883

(56) Entgegenhaltungen:
- EP-A1- 2 675 403
- EP-A1- 2 675 404
- WO-A1-2011/066960
- DE-A1-102009 006 180

## Beschreibung

Die Erfindung bezieht sich auf eine medizinische Vorrichtung zur Einfuhr in ein Hohlorgan, gemäß dem Oberbegriff des Patentanspruchs 1. Eine derartige medizinische Vorrichtung ist beispielsweise aus DE 101 27 602 A1 bekannt.

Für die Behandlung von Aneurysmen, von Stenosen und für die Aufweitung von verengten Gefäßen sind Vorrichtungen mit einer Gitterstruktur geeignet, die eine erhöhte Feinmaschigkeit aufweisen. Durch die Feinmaschigkeit können die Strömungsverhältnisse in einem Aneurysma beeinflusst werden, insbesondere durch eine Verlangsamung der Strömung im Aneurysma. Damit wird das Ziel verfolgt, die Gerinnung und Verödung des Aneurysmas zu bewirken. Die feinmaschige Gitterstruktur fördert zudem das schnelle Wachstum von Zellen, also die Endothelialisierung. Bei Vorrichtungen, die zur Aufweitung von Stenosen oder verengten Gefäßen eingesetzt werden, ermöglicht die Feinmaschigkeit außerdem ein Blockieren von abgetrennten Partikeln.

Eine erhöhte Feinmaschigkeit weisen insbesondere Gittergeflechte auf, die aus mehreren, sich kreuzenden Drähten gebildet sind. Ferner kommen Gittergeflechte bei der endovaskulären Behandlung vorteilhaft zum Einsatz, wenn eine erhöhte Flexibilität erforderlich ist. Beispielsweise können Filter oder Thrombenfänger, die in Blutgefäßen eingesetzt werden, in Form eines Gittergeflechts hergestellt sein. Die gute Flexibilität von Gittergeflechten ermöglicht die Behandlung auch in stark gewundenen Gefäßabschnitten, die beispielsweise im Bereich des Gehirns häufig vorkommen.

Es ist aus der Praxis bekannt, Gittergeflechte mit einer offenen Struktur herzustellen. Dabei weisen die Drähte des Geflechtes an den Geflechtenden freie Drahtenden auf. Derartige Gittergeflechte sind einfach und in hoher Stückzahl herstellbar, da die Drähte in einem Stück als langer Strang zu einem langgezogenen Gittergeflecht geflochten werden können, der in mehrere Gittergeflechte geeigneter Länge unterteilt wird. Dadurch ergeben sich verschiedene Einheiten mit jeweils offenen bzw. freien Drahtenden.

Alternativ können Geflechte mit atraumatisch wirkenden Geflechtenden hergestellt werden. Dabei werden die Drähte an den Geflechtenden entweder miteinander verbunden oder umgelenkt und in das Geflecht zurückgeführt. In beiden Fällen entstehen an wenigstens einem Geflechtende Schlaufen, die das Risiko einer Verletzung der Gefäßwand verringern.

Ein Beispiel für ein Gittergeflecht mit einem derart atraumatischen Geflechtende ist in der eingangs genannten DE 101 27 602 A1 offenbart. Darin ist ein Stent zur Implantation im menschlichen Körper mit einem radial komprimierbaren und expandierbaren Gittergeflecht beschrieben. An den Geflechtenden sind die freien Enden der Drähte des Gittergeflechts zusammengeführt und derart verbunden, dass an den Geflechtenden Schlaufen gebildet sind. Die Schlaufen an den Geflechtenden führen zu einer Abschlusskante des Geflechts mit einer gezackten Kontur. Konkret weist die Abschlusskante des bekannten Gittergeflechts eine Profilierung auf, wobei mehrere Profilabschnitte gebildet sind. Ein Profilabschnitt entspricht dabei einer Zacke der gezackten Abschlusskante.

Der Profilabschnitt ist durch insgesamt vier Drähte gebildet, wobei zwei Drähte innerhalb des Gittergeflechts eine erste Spiralrichtung und zwei weitere Drähte eine zweite Spiralrichtung bezogen auf die Längsachse des Gittergeflechts aufweisen. Die Drähte sind jeweils in Kreuzungsendpunkten des Gittergeflechts umgelenkt, zusammengeführt und verdrillt, um eine Spitze des Profilabschnitts zu bilden. Die beiden äußeren der vier Drähte bilden die Abschlusskante des Gittergeflechts bzw. zwei Abschnittskanten des Profilabschnitts.

Bei dem bekannten Stent weisen die Abschnittskanten der Profilabschnitte jeweils einen einzigen Draht auf. Dies geht zu Lasten der Stabilität der Abschlusskante. Überdies weist der bekannte Stent im Bereich der Abschlusskante, also am Geflechtende, eine geringere Radialkraft als innerhalb des Gittergeflechts auf. Somit besteht das Risiko, dass der bekannte Stent im expandierten Zustand eine zigarrenartige Form einnimmt. Dabei liegen sich die Stentenden bei der Implantation in einem Gefäß nicht ausreichend stark an die Gefäßwand an, so dass die Geflechtenden in den Strömungskanal des Gefäßes hineinragen. Das kann zu einem erhöhten Thrombogenisierungsrisiko führen.

Weitere medizinische Vorrichtungen sind beispielhaft aus DE 10 2009 006 180 A1 bekannt. Hier werden Geflechtenden in die Abschnittskante überführt, derart, dass das Gittergeflecht im Bereich der Abschnittskante verstärkt ausgebildet ist.

Die Aufgabe der Erfindung besteht darin, eine medizinische Vorrichtung, insbesondere zum Entfernen von Konkrementen aus Körperhohlorganen, mit einem komprimierbaren und expandierbaren Gittergeflecht anzugeben, das über die gesamte Länge eine ausreichende Radialkraft bereitstellt, um die Vorrichtung gleichmäßig, insbesondere zylinderförmig, zu expandieren und mit geringem Risiko für medizinische Komplikationen in einem Körperhohlgefäß zu positionieren.

Diese Aufgabe wird erfindungsgemäß durch den Gegenstand des Patentanspruchs 1 und im Rahmen eines nebengeordneten Aspekts durch den Gegenstand des Patentanspruchs 11 gelöst.

Die Erfindung beruht auf dem Gedanken, eine medizinische Vorrichtung, insbesondere zum Entfernen von Konkrementen aus Körperhohlorganen, mit einem komprimierbaren und expandierbaren Gittergeflecht anzugeben. Das Gittergeflecht umfasst eine Vielzahl erster Drähte und eine Vielzahl zweiter Drähte. Die Drähte sind jeweils spiralförmig um eine gemeinsame Längsachse gewunden und weisen unterschiedliche Spiralrichtungen auf, so dass sich die ersten Drähte und die zweiten Drähte zur Bildung von Maschen kreuzen. Das Gittergeflecht weist eine profilierte, umlaufende Abschlusskante auf. Die Abschlusskante umfasst mehrere Profilabschnitte mit jeweils glatten Abschnittskanten. Die Abschnittskanten erstrecken sich in unterschiedliche Spiralrichtungen entlang des Profilabschnitts und konvergieren zu einer Spitze des Profilabschnitts.

Die Abschnittskanten sind durch jeweils wenigstens zwei Drähte gebildet, von denen jeweils wenigstens ein Draht beim Übergang vom Gittergeflecht in die Abschlusskante an einer Umlenkstelle der jeweiligen Abschnittskante umgelenkt ist, so dass sich der umgelenkte Draht entlang der Abschnittskante in eine andere Spiralrichtung als innerhalb des Gittergeflechts erstreckt. Der umgelenkte Draht ist ein Einzeldraht, der nur an der jeweiligen Abschnittskante der Abschlusskante umgelenkt ist. Die beiden zur Spitze konvergierenden Abschnittskanten weisen jeweils eine, der Spitze entlang der Abschnittskante unmittelbar nachgeordnete, erste Umlenkstelle auf, an der ein Draht umgelenkt und der Abschnittskante zugeführt ist, so dass sich die Anzahl der Drähte jeder Abschnittskante jeweils an den Umlenkstellen bzw. an der Umlenkstelle ändert, insbesondere in Richtung zur Spitze des Profilabschnitts erhöht.
Erfindungsgemäß werden die Abschnittskanten durch jeweils wenigstens zwei Drähte gebildet. Die Stabilität der Abschnittskanten und somit allgemein der Abschlusskante wird auf diese Weise erhöht. Überdies wird dadurch die Expansion der Abschlusskante verbessert. Insbesondere die Integration mehrerer Drähte in eine Abschnittskante führt zu einer Verstärkung der Abschlusskante, so dass das Geflechtende insgesamt eine hohe Stabilität und Radialkraft aufweist. Ein wesentlicher Gedanke der Erfindung besteht darin, dass der Draht an einer Umlenkstelle umgelenkt wird, die in der Abschnittskante angeordnet ist. Innerhalb des Gittergeflechts verlaufen die Drähte also im Wesentlichen unverändert in ihrer vorgegebenen Spiralform. Das gewährleistet eine gleichmäßige Expansion des Gittergeflechts. Zur Bildung der umlaufenden Abschlusskante wird für jede Abschnittskante ein Draht umgelenkt und ändert somit seine Spiralrichtung, um der Abschnittskante zu folgen. Auf beiden Seiten der Spitze des Profilabschnitts ändern die in die jeweilige Abschnittskante mündenden Drähte ihre Spiralrichtung. Auf diese Weise wird erreicht, dass das Gittergeflecht einerseits gleichmäßig expandiert und andererseits die Abschlusskante des Gittergeflechts stabilisiert wird und der Expansion des Gittergeflechts gut folgt. Ferner wird durch die erfindungsgemäße Konstruktion erreicht, dass sich die Spitzen unmittelbar benachbarter Profilabschnitt der Abschlusskante im Wesentlichen gleichmäßig voneinander entfernen, ohne dass sich Versatz einzelner Spitzen in axialer Richtung einstellt. Dies ist beispielsweise bei den einzeln zusammengeführten und verdrillten Drahtenden gemäß DE 101 27 602 A1 nicht gewährleistet.

Die Umlenkung des einen Drahtes beim Übergang vom Gittergeflecht in die Abschlusskante an einer Umlenkstelle der jeweiligen Abschnittskante bedeutet, dass die Abschlusskante bzw. die jeweilige Abschnittskante der Abschlusskante direkt vom Gittergeflecht bzw. direkt aus dem Gittergeflecht gebildet ist. Der Übergang erfolgt ohne die Bildung von Zwischenformen des Gittergeflechts, wie vorgeordnete Geflechtenden. Die Gitterstruktur erstreckt sich ohne eine Umkehr der Spiralrichtung, also ohne eine Änderung der Spiralrichtung im Uhrzeiger- bzw. Gegenuhrzeigersinn direkt bis zur Abschlusskante bzw. zur jeweiligen Abschnittskante der Abschlusskante. Erst in bzw. an der Abschlusskante bzw. Abschnittskante erfolgt die Änderung der Spiralrichtung an der jeweiligen Umlenkstelle. Die Umlenkung des Drahtes erfolgt also nur an der jeweiligen Abschnittskante der Abschlusskante. Dabei bedeutet die Umlenkung an der jeweiligen Abschnittskante, dass die Umlenkstelle im Bereich, insbesondere auf der Abschnittskante liegt.

Die Abschnittskanten weisen vorzugsweise jeweils mehrere Umlenkstellen auf, an denen jeweils ein Draht umgelenkt und der Abschnittskante zugeführt ist derart, dass sich die Anzahl der Drähte jeder Abschnittskante jeweils an den Umlenkstellen ändert, insbesondere in Richtung zur Spitze des Profilabschnitts erhöht. Mit anderen Worten summiert sich die Anzahl der Drähte entlang einer Abschnittskante, vorzugsweise in Richtung zur Spitze des Profilabschnitts. Insbesondere weist die Abschnittskante mehrere Umlenkstellen auf, an denen jeweils ein weiterer Draht der Abschnittskante zugeführt wird. Dabei werden die einzelnen Drähte an den Umlenkstellen jeweils in Richtung zur Spitze des Profilabschnitts umgelenkt. Somit erhöht sich die Anzahl der Drähte entlang der Abschnittskante von Umlenkstelle zu Umlenkstelle in Richtung zur Spitze. Die Abschnittskanten der Profilabschnitte werden zur Spitze hin sukzessive verstärkt bzw. weisen eine sich sukzessive erhöhende Stabilität auf. Im Bereich der Spitze der Profilabschnitte liegt die Maximalanzahl von Drähten vor. Konkret umfasst die Spitze des Profilabschnitts wenigstens vier Drähte, wobei jeweils zwei Drähte einer Abschnittskante zugeordnet sind. Die Anzahl der Drähte in der Spitze des Profilabschnitts entspricht also der Summe der Drähte, die die Abschnittskanten dieses Profilabschnitts bilden. Bei zwei Abschnittskanten pro Profilabschnitt entspricht die Anzahl der Drähte in der Spitze des Profilabschnitts der doppelten Drahtanzahl je Abschnittskante.

Erfindungsgemäß ist weiterhin vorgesehen, dass der umgelenkte Draht ein Einzeldraht ist. Konkret ist an den Umlenkstellen bzw. der Umlenkstelle der jeweiligen Abschnittskante jeweils ein Einzeldraht in die Abschnittskante überführt. Der Einzeldraht ist von benachbarten Einzeldrähten im Gittergeflecht vor der Umlenkstelle beabstandet, insbesondere um eine Maschenbreite versetzt. Dadurch werden einerseits ein besonders feinmaschiges Geflecht und andererseits eine gute Crimpbarkeit der Vorrichtung erreicht.

Der Flechtwinkel des Einzeldrahts vor der Umlenkstelle kann bei einem bevorzugten Ausführungsbeispiel dem Flechtwinkel des Einzeldrahtes nach der Umlenkstelle entsprechen. Dadurch wird erreicht, dass die Abschlusskante symmetrisch zu den im Geflecht angeordneten Drähten verläuft. Der Umlenkwinkel, also der Winkel zwischen dem Draht vor und nach der Umlenkstelle kann beispielsweise 90° betragen. Andere Umlenkwinkel sind in Abhängigkeit vom jeweiligen Flechtwinkel möglich. Die Beibehaltung des Flechtwinkels vor und nach der Umlenkstelle vermeidet bzw. verringert Verzerrungen im Geflecht beim Komprimieren oder Expandieren.

Bei einem weiteren bevorzugten Ausführungsbeispiel erstreckt sich die Abschnittskante auf beiden Seiten der Umlenkstelle, wobei die Abschnittskante vor dem umgelenkten Einzeldraht aus mindestens zwei Drähten gebildet ist. Dies bedeutet, dass der umzulenkende Draht bzw. Einzeldraht im Bereich der bereits bestehenden bzw. bereits ausgebildeten Abschlusskante zugeführt wird. Da die Abschnittskante vor dem umgelenkten Einzeldraht aus mindestens zwei Drähten gebildet ist, weist diese eine erhöhte Stabilität auf.

Die beiden Drähte der Abschnittskante können vor dem ersten umgelenkten Einzeldraht tangential zusammengeführt sein. Dadurch wird die Herstellung des Geflechts insbesondere im Bereich der Abschnittskante erleichtert.

Die Zellen der Gitterstruktur können symmetrisch entlang der Abschlusskante bzw. der jeweiligen Abschnittskante der Abschlusskante angeordnet sein. Die Zellen können direkt in die Abschlusskante bzw. in die jeweilige Abschnittskante der Abschlusskante übergehen.

Bei einer bevorzugten Ausführung sind die in der Spitze zusammenlaufenden Drähte miteinander verdrillt und bilden einen Fortsatz, der sich im Wesentlichen parallel zur Längsachse des Gittergeflechts erstreckt. Durch die Verdrillung wird eine einfache Möglichkeit geschaffen, die Drahtenden der einzelnen Drähte, die die Abschnittskanten bzw. allgemein die Abschlusskante bilden, miteinander zu verbinden. Obwohl mit den verdrillten Drahtenden ein atraumatisches Geflechtende gebildet ist, besteht bei der Konstruktion die Möglichkeit, das Gittergeflecht kontinuierlich als langen Strang herzustellen und anschließend in einzelne Einheiten zu unterteilen. Dazu kann das Gittergeflecht auf einer Flechtmaschine geflochten werden, wobei in vorbestimmten Abständen die Abschlusskante durch entsprechendes Umlenken von Drähten gebildet wird. Die in den Spitzen der Profilabschnitte zusammengeführten Drähte werden miteinander verdrillt und im weiteren Verlauf wieder aufgefächert, um in ein neues Gittergeflecht überführt zu werden. Der damit gebildete lange Gittergeflechtsstrang kann im Bereich der Fortsätze auseinander geschnitten werden, um einzelne Einheiten von Gittergeflechten zu bilden. Das Verdrillen der einzelnen Geflechtenden zur Bildung der Fortsätze hat den weiteren Vorteil, dass durch die Verbindung der Drähte eine verbesserte Expansion der Abschlusskante bzw. allgemein des Geflechtendes erreicht wird. Insbesondere wird durch die Verbindung der Drähte miteinander eine erhöhte Radialkraft im Bereich der Abschlusskante bereitgestellt. Die Bildung einer Zigarrenform des Gittergeflechts im expandierten Zustand wird somit vermieden.

Der Fortsatz kann wenigstens ein Verbindungselement, insbesondere eine Hülse, und/oder ein Markerelement, insbesondere einen Röntgenmarker, aufweisen. Das Verbindungselement kann als zusätzliche Fixierung der Drahtenden in den Fortsätzen genutzt werden. Beispielsweise kann das Verbindungselement als crimpbare Hülse ausgebildet sein, so dass die im Fortsatz verdrillten Drahtenden zusätzlich kraftschlüssig durch die Crimphülse fixiert sind. Die Hülse kann mit den verdrillten Drähten im Fortsatz verschweißt sein bzw. durch Schweißen mit den Drähten verbunden sein. Es ist auch möglich, dass die Drahtenden im Bereich des Fortsatzes nicht verdrillt sind, sondern parallel zueinander verlaufen. Die Fixierung der Drahtenden erfolgt dann vorzugsweise durch eine derartige Crimphülse. Die Hülse kann gleichzeitig ein röntgendichtes Material aufweisen, so dass die Fortsätze, die das Geflechtende markieren, unter Röntgenkontrolle gut erkennbar sind. Die Hülse kann also auch ein Markerelement bilden. Andere Markerelemente, die eine verbesserte Sichtbarkeit der Fortsätze bzw. allgemein des Geflechtendes, insbesondere unter Einfluss von Röntgenstrahlung, ermöglichen, können eingesetzt werden. Beispielsweise können die Fortsätze der einzelnen Profilabschnitte durch verdrillte Drahtenden gebildet sein, wobei zusätzlich ein Draht in die Verdrillung integriert ist, der eine erhöhte Röntgensichtbarkeit aufweist. Eine weitere Möglichkeit besteht darin, einen Stift aus einem röntgensichtbaren Material in den Fortsatz zu integrieren, wobei die verdrillten Drahtenden der Geflechtdrähte den Stift einhüllen. Mit anderen Worten können die Drahtenden im Bereich des Fortsatzes derart verdrillt sein, dass ein röntgensichtbarer Stift von den Drahtenden umschlungen ist.

Das Geflecht kann einen oder mehrere Verstärkungsdrähte aufweisen, der dicker als die übrigen Drähte ist. Vorzugsweise weist der Verstärkungsdraht einen Querschnittsdurchmesser auf, der um wenigstens 50 %, insbesondere wenigstens 100 %, größer als der Querschnittsdurchmesser der übrigen Drähte ist. Im Allgemeinen erhöht der Verstärkungsdraht die Stabilität des Gittergeflechts, wobei sich die vorgenannten Querschnittsdurchmesserverhältnisse für die insgesamt relativ kleinen Dimensionen der erfindungsgemäßen Vorrichtung als besonders vorteilhaft zur Erhöhung der Stabilität erwiesen haben.

Im Rahmen der Anmeldung sind die Drähte , die unterschiedliche Spiralrichtungen aufweisen, nicht als streng voneinander getrennte Bauteile zu verstehen. Vielmehr können die Drähte auch Drahtabschnitte desselben Drahts bilden, der in unterschiedlichen Spiralrichtungen gewunden ist, um das Gittergeflecht zu bilden.

Bei einer bevorzugten Ausführungsform der erfindungsgemäßen Vorrichtung sind beide Drähte, insbesondere alle Drähte, die gemeinsam eine Abschnittskante bilden, beim Übergang vom Gittergeflecht in die Abschlusskante an jeweils einer Umlenkstelle umgelenkt. Mit anderen Worten können die Abschnittskanten jeweils ausschließlich durch Drähte gebildet sein, die umgelenkt sind und entlang der Abschnittskante bzw. allgemein der Abschlusskante eine andere Spiralrichtung aufweisen als im Gittergeflecht. Beispielsweise können alle Drähte des Gittergeflechts, die innerhalb des Gittergeflechts im Uhrzeigersinn um eine gemeinsame Längsachse spiralförmig gewunden sind, im Bereich der Abschlusskante umgelenkt sein und Drahtenden bilden, die entlang der jeweiligen Abschnittskanten im Gegenuhrzeigersinn verlaufen. Umgekehrt sind in diesem Fall alle Drähte des Gittergeflechts, die innerhalb des Gittergeflechts im Gegenuhrzeigersinn um eine gemeinsame Längsachse spiralförmig gewunden sind, im Bereich der Abschlusskante zur Bildung der einzelnen Abschnittskanten im Uhrzeigersinn gewunden.

Alternativ kann vorgesehen sein, dass die Abschnittskanten der Profilabschnitte jeweils einen Draht aufweisen, der in der Abschnittskante dieselbe Spiralrichtung wie im Gittergeflecht aufweist. Jede Abschnittskante kann also durch einen Draht gebildet sein, der aus dem Gittergeflecht kommend unter Beibehaltung seiner Spiral richtung in die Abschlusskante überführt wird. Vorzugsweise bildet der Draht, der in der Abschlusskante dieselbe Spiralrichtung wie im Gittergeflecht aufweist, in dieser Konfiguration einen kantenbildenden Draht der Abschlusskante, dem an einer oder mehreren Umlenkstellen der Abschnittskante wenigstens ein weiterer Draht oder mehrere weitere Drähte zugeführt werden, die im Gittergeflecht eine gegenläufige Spiralrichtung aufweisen und umgelenkt sind, um der Spiralrichtung des ersten Drahts zu folgen. Durch den kantenbildenden Draht der Abschnittskante, der beim Übergang vom Gittergeflecht in die Abschnittskante seine Spiralrichtung beibehält, wird erreicht, dass die jeweils ersten Drähte von unmittelbar benachbarten Abschnittskanten unmittelbar benachbarter Profilabschnitte zwischen den unmittelbar benachbarten Profilabschnitten einen Kreuzungspunkt bilden. Die kantenbildenden Drähte zweier Abschnittskanten unmittelbar benachbarter Profilabschnitte überkreuzen sich also an einem Punkt, der die beiden Profilabschnitte voneinander trennt. Dadurch wird die Stabilität der Abschlusskante insgesamt erhöht und die Expansion der Abschlusskante verbessert.

Vorteilhaft kann außerdem vorgesehen sein, dass die Drähte entlang einer Abschnittskante, insbesondere an den Übergangsstellen, miteinander verbunden sind. Beispielsweise können die Drähte zwischen zwei Übergangsstellen verdrillt sein. Dadurch ist gewährleistet, dass die Abschlusskante in jedem Expansionsstadium eine glatte Abschlusskante bildet. Überdies wird auf diese Weise die Radialkraft der Abschlusskante erhöht.

Gemäß einem nebengeordneten Aspekt beruht die Erfindung auf dem Gedanken, eine medizinische Vorrichtung, insbesondere zum Entfernen von Konkrementen aus Körperhohlorganen, mit einem komprimierbaren und expandierbaren Gittergeflecht anzugeben, das eine profilierte, umlaufende Abschlusskante aufweist. Die Abschlusskante umfasst mehrere Profilabschnitte mit jeweils einer glatten, abgerundeten Abschnittskante. Die Abschnittskante ist aus wenigstens zwei Drähten gebildet, die sich entlang der Abschnittskante überlappende Schlaufen bilden und in einem Scheitelpunkt der Abschnittskante verbunden sind. Der Scheitelpunkt der Abschnittskante unterteilt die Drähte in jeweils einen ersten Drahtabschnitt und einen zweiten Drahtabschnitt. Der erste Drahtabschnitt und der zweite Drahtabschnitt sind in jeweils unterschiedlichen Spiralrichtungen spiralförmig um eine gemeinsame Längsachse gewunden derart, dass sich der erste Drahtabschnitt und der zweite Drahtabschnitt zur Bildung von Maschen im Gittergeflecht kreuzen.

Dieser Aspekt der Erfindung geht auf die Idee zurück, das Geflechtende aus Schlaufen aufzubauen, die jeweils aus einem Draht gebildet sind, der in einer ersten Spiralrichtung verlaufend aus dem Gittergeflecht kommt, im Bereich der Abschlusskante umgelenkt wird und in eine zweite Spiralrichtung, insbesondere eine gegenläufige Spiralrichtung, verlaufend in das Gittergeflecht zurückgeführt ist. Der im Bereich der Abschlusskante umgeschlaufte Draht weist also zwei Drahtabschnitte auf, die innerhalb des Gittergeflechts in unterschiedlichen Spiralrichtungen verlaufen. Die beiden Drahtabschnitte kreuzen sich daher in unmittelbarer Nähe zur Abschlusskante. Um die Stabilität der Abschlusskante zu erhöhen und die Expansion der Abschlusskante zu verbessern, ist erfindungsgemäß vorgesehen, dass mehrere Drähte, insbesondere wenigstens zwei Drähte, jeweils eine Schlaufe bilden, wobei die Schlaufen der Drähte sich zumindest entlang der Abschnittskante eines Profilabschnitts überlappen und zumindest im Scheitelpunkt des Profilabschnitts bzw. der Abschnittskante miteinander verbunden sind. Die Schlaufen der Drähte eines Profilabschnitts können unterschiedlich groß sein, wobei die Schlaufen zumindest im Scheitelpunkt der Abschnittskante aufeinander angeordnet und miteinander verbunden sind. Der Vorteil dieses Erfindungsaspekts besteht darin, dass eine im Wesentlichen wellenförmige Abschlusskante bereitgestellt wird, die besonders atraumatische Eigenschaften aufweist.

Die Erfindung wird im Folgenden anhand von Ausführungsbeispielen unter Bezugnahme auf die beigefügten, schematischen Zeichnungen näher erläutert. Darin zeigen:
- Fig. 1:: eine Abwicklung eines Gittergeflechts nach einem bevorzugten Ausführungsbeispiel der erfindungsgemäßen medizinischen Vorrichtung;
- Fig. 1a: das Ausführungsbeispiel nach Fig. 1 mit Umlenkstiften zur Herstellung des Geflechts;
- Fig. 1b: eine Abwicklung eines Gittergeflechts nach einem weiteren bevorzugten Ausführungsbeispiel mit Verstärkungsdrähten;
- Fig. 1c: eine Abwicklung eines Gittergeflechts nach einem weiteren bevorzugten Ausführungsbeispiel mit Verstärkungsdrähten und Mehrfachumlenkungen pro Umlenkstift;
- Fig. 1d: eine Abwicklung eines Gittergeflechts nach einem bevorzugten Ausführungsbeispiel mit Mehrfachumlenkungen pro Umlenkstift, wobei pro Abschnittskante ein einziger Umlenkstift vorgesehen ist;
- Fig. 1e: einen vergrößerten Ausschnitt aus Fig. 1d;
- Fig. 1f: eine Abwicklung eines Gittergeflechts nach einem weiteren bevorzugten Ausführungsbeispiel mit zwei Umlenkstiften pro Abschnittskante;
- Fig. 2:: eine Abwicklung eines Gittergeflechts der erfindungsgemäßen medizinischen Vorrichtung gemäß einem Ausführungsbeispiel mit einer aus Schlaufen gebildeten Abschlusskante.

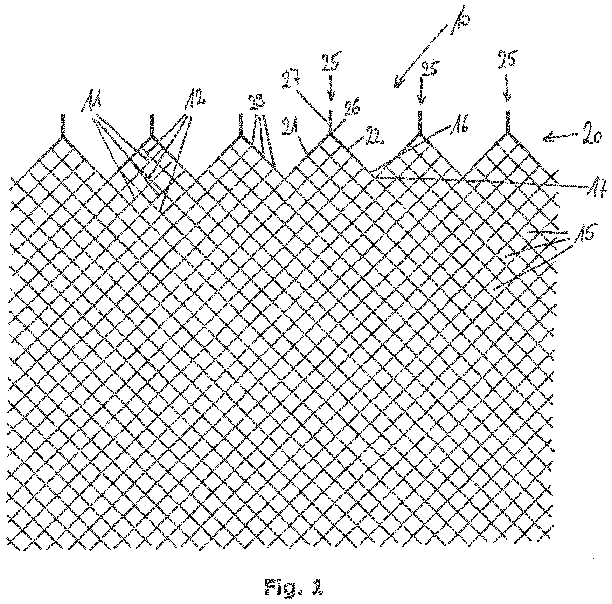

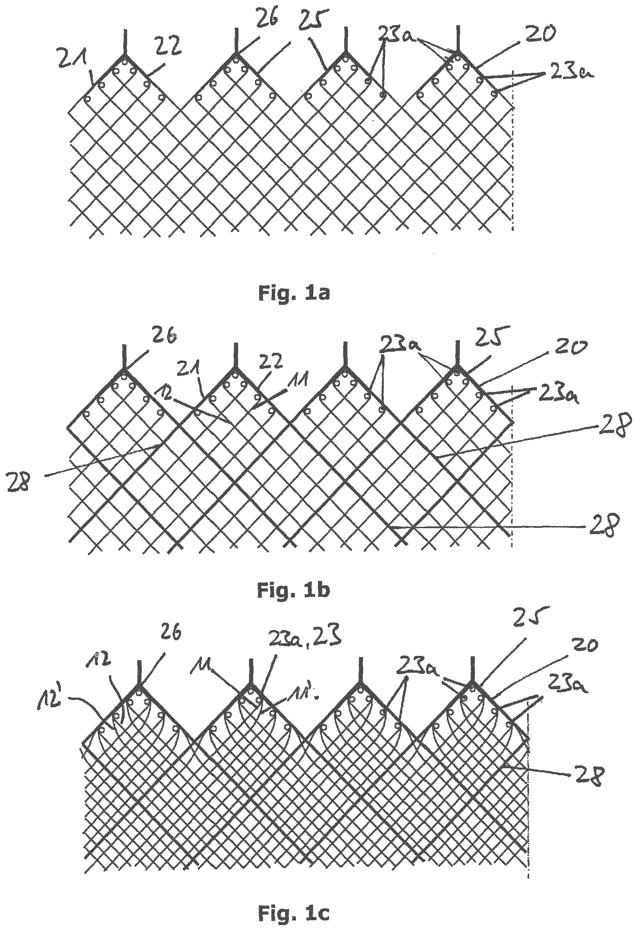

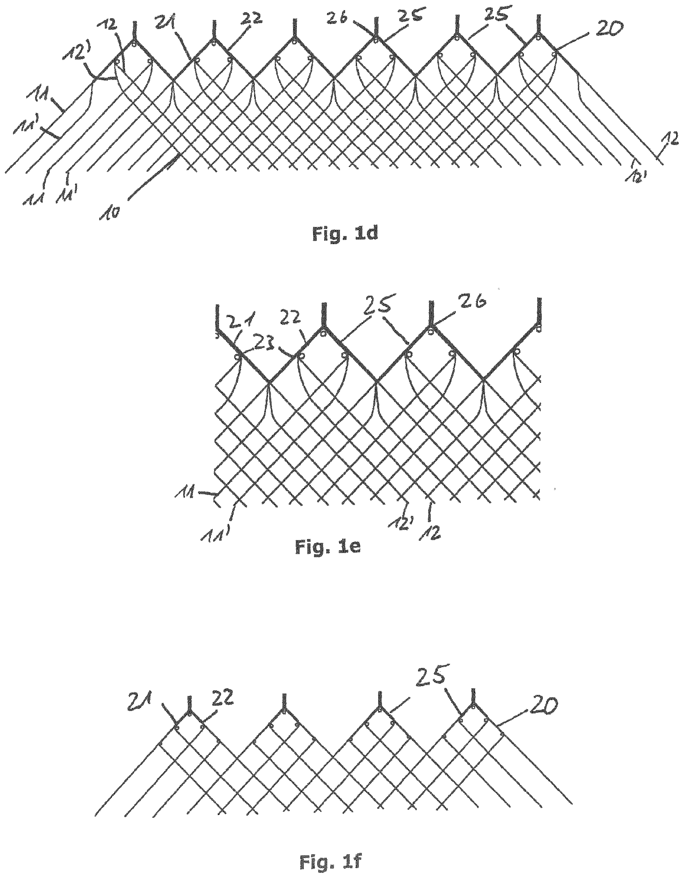

In Fig. 1 ist eine Abwicklung eines Gittergeflechts 10 dargestellt. Das Gittergeflecht 10 bildet im Gebrauch einen Hohlraum mit einem Lumen, insbesondere einen länglichen Hohlraum der eine axiale Erstreckung aufweist und von einer in Umfangsrichtung gekrümmten Wandung begrenzt ist. Die Wandung ist durch das Gittergeflecht 10 gebildet.

Die aus dem Gittergeflecht gebildete medizinische Vorrichtung ist zur Behandlung von Stenosen und/oder Aneurysmen, beispielsweise in interkranialen Gefäßen, in der Carotis, im Bauchraum bzw. in Bauchorganen, in Gefäßen im Bereich der Beine usw. geeignet. Die Vorrichtung kann ein Implantat, insbesondere ein Stent, oder ein temporär in den Körper einführbares medizinisches Element, insbesondere ein Rekanalisationselement oder ein Thrombenfänger sein.

Das Gittergeflecht 10 umfasst mehrere Drähte 11, 12, wobei eine Vielzahl erster Drähte 11 eine erste Spiralrichtung und eine Vielzahl zweiter Drähte 12 eine zweite Spiralrichtung aufweisen. Die erste und die zweite Spiralrichtung sind unterschiedlich, insbesondere gegenläufig. Konkret bildet das Gittergeflecht 10 einen rohrförmigen, insbesondere zumindest abschnittsweise kreiszylindrischen Körper, wobei die ersten Drähte 11 und die zweiten Drähte 12 in gegenläufiger Spiralrichtung um eine gemeinsame Längsachse des Gittergeflechts 10 gewunden sind. In der Zeichnungsebene gemäß Fig. 1 verlaufen die ersten Drähte 11 von links unten nach rechts oben und die zweiten Drähte 12 von rechts unten nach links oben.

Das Gittergeflecht 10 weist eine Abschlusskante 20 auf, die profiliert ausgebildet ist. Die profilierte Abschlusskante 20 ist insbesondere zickzack- bzw. kronenförmig gebildet. Insgesamt weist die Abschlusskante 20 also eine kronenartige Struktur auf. Die einzelnen Zacken der kronenartigen Struktur der Abschlusskante 20 werden im Rahmen der Anmeldung als Profilabschnitte 25 bezeichnet. Die Abschlusskante 20 weist wenigstens zwei Profilabschnitte 25 auf. Die Profilabschnitte 25 umfassen jeweils zwei Abschnittskanten 21, 22, die jeweils eine Flanke der zackenförmigen Profilabschnitte 25 bilden. Die Abschnittskanten jedes Profilabschnitts 25 konvergieren zu einer Spitze 26. Mit anderen Worten weist jeder Profilabschnitt 25 eine Spitze 26 auf, in der die Abschnittskanten 21, 22 des Profilabschnitts 25 miteinander verbunden sind.

Die Abschnittskanten 21, 22 sind jeweils durch wenigstens zwei Drähte 11, 12 gebildet. Vorzugsweise sind die Abschnittskanten 21, 22 durch jeweils drei oder mehr Drähte, bei dem Ausführungsbeispiel gemäß Fig. 1 durch vier Drähte 11, 12 gebildet. Wenigstens einer der Drähte 11, 12 erstreckt sich über die gesamte Länge der Abschnittskante 21, 22. Die weiteren Drähte der Abschnittskante 21, 22 verlaufen über einen Teil der Abschnittskante 21, 22.

Die Abschnittskanten 21, 22 erstrecken sich jeweils in eine Spiralrichtung der Drähte 11, 12. Dabei weisen die Abschnittskanten 21, 22 jeweils eine unterschiedliche Spiralrichtung auf. Die Spiralrichtung der Abschnittskanten 21, 22 entspricht also jeweils der Spiralrichtung einer Vielzahl der Drähte 11, 12 im Gittergeflecht 10. Die Kontur und/oder die Länge der Abschnittskanten 21, 22 wird im Wesentlichen durch einen einzigen Draht 11, 12 vorgegeben, der sich über die gesamte Länge der Abschnittskante 21, 22 erstreckt. Bei dem Ausführungsbeispiel gemäß Fig. 1 wird der Verlauf der Abschnittskanten 21, 22 jeweils durch einen Draht 11, 12 bestimmt, der in der Abschnittskante 22 dieselbe Spiralrichtung aufweist wie im Geflecht 10. Konkret weisen die Abschnittskanten 21, 22 jeweils einen kantenbildenden Draht 16 auf, der aus dem Geflecht 10 kommend ohne Änderung der Spiralrichtung in die Abschlusskante 21, 22 übergeht. Zwischen den einzelnen Profilabschnitten 25 kreuzen sich jeweils zwei kantenbildende Drähte 16 und bilden einen Kreuzungsendpunkt 17, der jeweils einen Endpunkt zweier Abschnittskanten 21, 22 benachbarter Profilabschnitte 25 bildet. Die Abschnittskanten 22 sind ferner durch die Spitze 26 des jeweiligen Profilabschnitts 25 begrenzt. Mit anderen Worten erstrecken sich die Abschnittskanten 21, 22 jeweils zwischen einem Kreuzungsendpunkt 17 und der Spitze 26.

Das Geflecht 10 bildet in axialer Richtung bis zum Kreuzungsendpunkt 17 eine durchgehende Wandung, insbesondere mit einer konischen und/oder zylindrischen Mantelfläche.

Zur Bildung der Abschnittskante 21, 22 ist dem kantenbildenden Draht 16, der in die Abschnittskante 21, 22 mit unveränderter Spiralrichtung übergeht, wenigstens ein weiterer Draht 11, 12 zugeführt, der an einer Umlenkstelle 23 der Abschnittskante 21, 22 umgelenkt wird und seine Spiralrichtung ändert, um der Spiralrichtung der Abschnittskante 21, 22 zu folgen.

Grundsätzlich gilt, dass der kantenbildende Draht 16 vor dem Übergang in die Abschnittskante 21, 22 nicht nur dieselbe Spiralrichtung, sondern auch denselben Flechtwinkel wie die Abschnittskante 21, 22 aufweist. Mit anderen Worten fluchten die Abschnittskante 21, 22 und der kantenbildende Draht 16. Der Winkel zwischen der Abschnittskante 21, 22 und der Längsachse des Gittergeflechts 10 bzw. einer Projektion der Längsachse in die Umfangsebene des Gittergeflechts 10 entspricht also dem Flechtwinkel des kantenbildenden Drahts 16.

Die Zuführung von weiteren Drähten 11, 12 zur Abschlusskante 21, 22 wird im Folgenden anhand einer Abschnittskante 21 erläutert, wobei Analoges auch für die Abschnittskante 22 gilt, die in eine gegenläufige Spiralrichtung verläuft:

Die Abschnittskante 21 umfasst einen kantenbildenden Draht 16, der im Wesentlichen durch einen ersten Draht 11 gebildet ist, der dieselbe Spiralrichtung wie die Abschnittskante 21 aufweist. Die Abschnittskante 21 ist also durch eine Fortsetzung eines ersten Drahts 11 gebildet, wobei dieser erste Draht 11 den kantenbildenden Draht 16 bildet. Dem kantenbildenden Draht 16 wird stufenweise an Umlenkstellen 23 jeweils ein weiterer zweiter Draht 12 zugeführt, wobei die zweiten Drähte 12 im Gittergeflecht 10 eine andere Spiralrichtung aufweisen als der erste Draht 11 bzw. kantenbildende Draht 16. Die Umlenkstellen 23 entlang der Abschnittskante 21 sind vorzugsweise regelmäßig beabstandet, weisen also im Wesentlichen denselben Abstand zueinander auf. An jeweils einer Umlenkstelle 23 wird ein zweiter Draht 12 dem kantenbildenden Draht 16 bzw. der Abschnittskante 21 zugeführt. Dabei wird der jeweilige zweite Draht 12 an der Umlenkstelle 23 umgelenkt und ändert seine Spiralrichtung, um der Spiralrichtung der Abschnittskante 21 zu folgen. Auf diese Weise erhöht sich die Drahtanzahl in der Abschnittskante 21 ausgehend vom Kreuzungsendpunkt 17 zur Spitze 26 hin an jedem Umlenkpunkt 23 um wenigstens 1. Zwischen dem letzten Umlenkpunkt 23 und der Spitze 26 ist die Drahtanzahl maximal. Zwischen den Umlenkpunkten 23 können die Drähte 11, 12 miteinander verdrillt sein. Vorzugsweise sind die Drähte zweier Abschnittskanten 21, die einen Profilabschnitt 25 bilden, also Drähte eines Profilabschnitts 25, im Bereich der Spitze 26 miteinander verdrillt und bilden einen Fortsatz 27. Der Fortsatz 27 erstreckt sich vorzugsweise im Wesentlichen parallel zur Längsachse des Gittergeflechts 10. Der Fortsatz 27 kann zur Aufnahme von Markerelementen, insbesondere Röntgenmarkern und/oder Hülsen, genutzt werden.

Alternativ zu der zuvor beschriebenen Variante, wobei der kantenbildende Draht 16 beim Übergang vom Gittergeflecht 10 in die Abschnittskante 21, 22 seine Spiralrichtung beibehält, kann vorgesehen sein, dass alle Drähte 11, 12 einer Abschnittskante 21, 22 im Geflecht 10 eine andere Spiralrichtung als in der Abschnittskante 21, 22 aufweisen. Beispielsweise kann die Abschnittskante 21 ausschließlich durch zweite Drähte 12 gebildet sein, die an den Umlenkstellen 23 der Abschnittskante 21 umgelenkt sind und der Spiralrichtung der Abschnittskante 21 folgen. Dabei kann ein kantenbildender Draht 16, der durch einen umgelenkten zweiten Draht 12 gebildet ist, im Kreuzungsendpunkt 17 mit einem weiteren kantenbildenden Draht 16 einer benachbarten Abschnittskante 21, 22 verschlungen sein. Andererseits kann vorgesehen sein, dass die umgelenkten kantenbildenden Drähte 16 benachbarter Abschnittskanten 21, 22 unterschiedlicher Profilabschnitte 25 keinen Kreuzungsendpunkt 17 bilden, also in diesem Bereich nicht miteinander verschlungen oder überkreuzt sind, sondern vielmehr derart umgelenkt sind, dass sich zwischen den beiden kantenbildenden Drähten 16 ein Spalt bildet. Dadurch wird die Flexibilität des Geflechtendes des Gittergeflechts 10 erhöht.

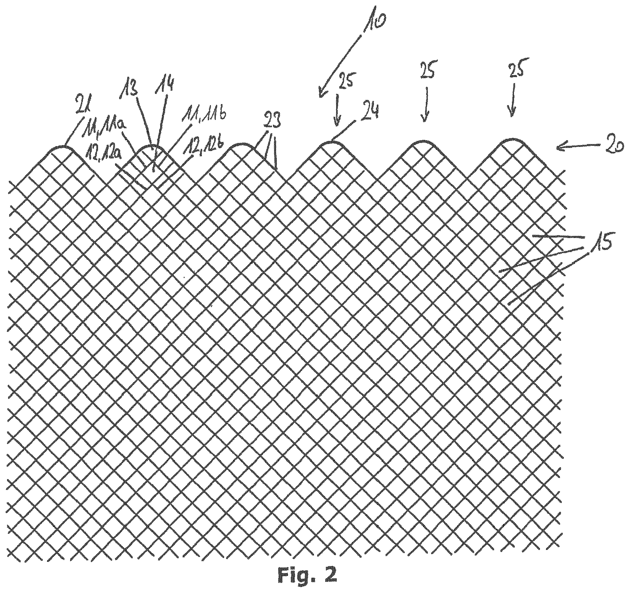

Die Gestaltung der Abschnittskanten 21, 22 durch sukzessive Zuführung weiterer Drähte 11, 12 und graduelle Erhöhung der Drahtanzahl innerhalb der Abschnittskanten 21, 22 ist in der auf die Anmelderin zurückgehenden und nachveröffentlichten deutschen Patentanmeldung 10 2009 056 450.0, insbesondere auf Seiten 15-23 in Verbindung mit den dortigen Figuren 2 und 3 detailliert erläutert. Der Offenbarungsgehalt der vorliegenden Anmeldung, zumindest aber der in Bezug genommenen Ausschnitte wird durch Verweis vollumfänglich in die vorliegende Anmeldung aufgenommen.

Generell wird offenbart, dass sich die Abschnittskanten 21, 22 jeweils zwischen der Spitze 26 und den Kreuzungsendpunkten bzw. allgemein einer Endüberkreuzung 17 der Drähte 11, 12 des Gittergeflechts erstrecken, die in axialer Richtung des Gittergeflechts 10 die letzte, in Umfangsrichtung geschlossene Reihe der Maschen 15 begrenzt, bevor die Drähte 11, 12 in die Profilabschnitte 25 übergehen. Die Kreuzungsendpunkte 17 bzw. Endüberkreuzungen der Drähte 11, 12 befinden sich in etwa auf derselben Höhe und bilden eine imaginäre Linie. Diese Linie grenzt das Gittergeflecht bzw. die Wandung der Vorrichtung von den Profilabschnitten 25 ab. Auf der Wandungsseite befindet sich die letzte in Umfangsrichtung geschlossene Reihe der Maschen 15. Auf der anderen Seite der Linie befindet sich die erste Maschenreihe der Profilabschnitt 25, die in Umfangsrichtung durch die jeweilige Abschnittskante 21, 22 unterbrochen ist. Die Maschen 15 des Gittergeflechts 10, das die in Umfangrichtung geschlossene Wandung der Vorrichtung bildet, erstrecken sich in Längsrichtung über die Kreuzungsendpunkte 17 bzw. über die letzte geschlossene Maschenreihe hinaus bis zur Spitze 26 des jeweiligen Profilabschnittes 25. Die Maschen 15 des Gittergeflechts 10 setzen sich also bis zur Spitze 26 des jeweiligen Profilabschnitts 25 fort, insbesondere gleichmäßig fort derart, dass die Profilabschnitte 25 durch das Gittergeflecht 10 gebildet sind. Damit wird erreicht, dass die Profilabschnitte 25 Wandungsabschnitte der Vorrichtung bilden, die durch die profilierte Abschlusskante 20 unterbrochen sind. Da das Gittergeflecht 10 im Bereich der Profilabschnitte 25 dem Gittergeflecht 10 im übrigen Bereich der Vorrichtung im Wesentlichen entspricht, werden Verzerrungen des Gittergeflechts beim Komprimieren bzw. Expandieren der Vorrichtung weitestgehend vermieden. Die Vorrichtung lässt sich daher besonders gut crimpen.

Ferner wird allgemein offenbart, dass sich die Maschen auf der Wandungsseite gleichmäßig, d.h. im Wesentlichen mit derselben Größe und/oder derselben Form über die Kreuzungsendpunkte 17 bzw. über die letzte, in Umfangsrichtung geschlossene Maschenreihe 15 hinaus in die Profilabschnitt 25 hinein erstrecken. Auf beiden Seiten der Kreuzungsendpunkte 17 liegt im Wesentlichen dieselbe Geflechtstruktur vor.

Die letzte Reihe der in Umfangsrichtung geschlossenen Maschen ist aus den Maschen gebildet, deren Kanten vollständig im Geflecht angeordnet sind, die also keinen Teil der Abschnittskante bilden. Die Drahtanzahl pro Masche ist im Profilabschnitt 25 konstant bis auf die äußeren Maschen, die direkt an die Abschlusskante 20 angrenzen und kantenbildende Drähte umfassen. Deren Anzahl verändert sich im Verlauf der Abschlusskante 20.

Im Allgemeinen erstreckt sich das Gittergeflecht 10 in die Profilabschnitte 25 hinein, insbesondere bis zu den Abschnittskanten 21, 22. Mit anderen Worten weist das Gittergeflecht 10 ein Geflechtmuster auf, das sich gleichmäßig bis zur Abschlusskante 20 fortsetzt. Die Profilabschnitte 25 umfassen also die gleichen oder zumindest gleichförmigen Maschen, die die letzte Maschenreihe der in Umfangsrichtung geschlossenen Maschen bilden. Die entfernt von der Abschlusskante 20 das Gittergeflecht 10 bildenden Maschen 15 setzen sich also zur Bildung der Profilabschnitte 25 fort und grenzen an die Abschlusskante 20 bzw. die Abschnittskanten 21, 22 an.

Das Gittergeflecht 10 im Bereich der Profilabschnitte 25 besteht aus Einzeldrähten, die an der Abschlusskante, insbesondere nur an der Abschlusskante umgelenkt sind. Die Einzeldrähte sind jeweils vom nächst benachbarten Draht 11, 12 des Gittergeflechts 10 beabstandet derart, dass der Einzeldraht bzw. umgelenkte Draht 11, 12 und der nächst benachbarte Draht 11, 12 die Maschen 15 des Gittergeflechts 10 zumindest teilweise begrenzen. Bei den Einzeldrähten handelt es sich also nicht um Drahtstränge, sondern um voneinander beabstandete einzelne Drähte.

Durch die Einzeldrähte wird erreicht, dass das Gittergeflecht feinmaschig aufgebaut werden kann. Außerdem wird die Crimpbarkeit der Vorrichtung verbessert.

Ferner wird allgemein offenbart, dass die Umlenkstelle 23, insbesondere die in Richtung der Spitze 26 gesehene erste Umlenkstelle 23 der jeweiligen Abschnittskante 21, 22 von der Endüberkreuzung bzw. dem Kreuzungsendpunkt 17 in Kantenrichtung um wenigstens eine Maschenbreite beabstandet ist derart, dass der Einzeldraht des Gittergeflechts 10 direkt in die jeweilige Abschnittskante zur Bildung der profilierten Abschlusskante 20 übergeht.

Die vorstehend beschriebenen Merkmale werden allgemein im Zusammenhang mit der Erfindung offenbart, ohne diese darauf einzuschränken.

Fig. 1a zeigt das Geflecht 10 gemäß Fig. 1, wobei an den Umlenkungen bzw. Umlenkstellen 23 der Abschlusskante 20 die zugehörigen Umlenkstifte 23a einer Fertigungshilfe, bspw. eines Flechtdorns, angeordnet sind. Die sukzessive der Abschlusskante 20 zugeführten Drähte 10, 11 liegen an den Umlenkstiften 23a tangential an und sind auf einem Teilumfang der Umlenkstifte 23a zur Änderung der Spiralrichtung geführt bzw. geformt.

Fig. 1b zeigt ein Geflecht 10, das zusätzlich zu den Merkmalen des Geflechts gemäß Fig. 1 Verstärkungsdrähte 28 aufweist, die für eine verbesserte Stabilität und erhöhte Radialkraft sorgen. Die Verstärkungsdrähte 28 fluchten mit den Abschnittskanten 21, 22. Die Verstärkungsdrähte 28 gehen aus dem Geflecht 10 kommend in die jeweilige Abschnittskante 21, 22 ohne Änderung der Spiralrichtung über. Die Verstärkungsdrähte 28 sind kantenbildende Drähte. Die Spiralrichtung der Abschnittskanten 21, 22 und der zugehörigen Verstärkungsdrähte 28, also der in die Abschnittskanten 21, 22 übergehenden Verstärkungsdrähte 28, sind gleich. Der Kreuzungsendpunkt 17 ist durch zwei sich überkreuzende Verstärkungsdrähte 28 gebildet und begrenzt die Abschlusskante 20 bzw. die jeweilige Abschnittskante 21, 22 in axialer Richtung.
Die aus dem Geflecht 10 kommenden Drähte 11, 12 gehen in der im Zusammenhang mit Fig. 1 beschriebenen Weise in die jeweiligen Abschnittskanten 21, 22 über und werden dabei umgelenkt, so dass die Drähte 11, 12 zusammen mit den Verstärkungsdrähten 28 die Abschlusskante bilden.

Die Verstärkungsdrähte 28 können eine größeren Drahtdurchmesser als die übrigen Drähte im Geflecht 10 aufweisen oder bspw. als Drahtstrang aus mehreren zusammengefassten Einzeldrähten ausgebildet sein. Dien Einzeldrähte pro Strang können parallel, also gerade nebeneinander, verdrillt, geflochten oder anderweitig zusammengefasst sein.

Generell können die Drähte metallisch oder aus einem anderen Material, bspw. aus Kunststoff gebildet sein. Die Drähte können aus einem Formgedächtniswerkstoff, bspw. aus Nitinol gefertigt sein.

In Fig. 1c ist ein Ausführungsbeispiel offenbart, das dem Ausführungsbeispiel gemäß Fig. 1b entspricht, wobei aus fertigungstechnischen Gründen ein Umlenkstift 23a zur Umlenkung mehrerer Einzeldrähte 11, 12 verwendet wird. Dies hat den Vorteil, dass sehr feinmaschige Geflechte 10 mit dem profilierten Geflechtabschluss herstellbar sind, da die Drahtabstände im Geflecht durch den Durchmesser und Abstand der Umlenkstifte 23a nicht begrenzt sind. Bspw. lässt sich die Drahtanzahl verdoppeln, wenn pro Umlenkstift 23a jeweils zwei Einzeldrähte 11, 12 umgelenkt werden. An einer Umlenkstelle 23 werden also wenigstens zwei Einzeldrähte 11, 12 umgelenkt. Bei Mehrfachumlenkungen pro Stift 23a vervielfacht sich die Drahtanzahl entsprechend. Bei der Umlenkung von wenigstens zwei Einzeldrähten 11, 12 wird einer der beiden Einzeldrähte 11, 12 an der Umlenkstelle 23 scharf umgelenkt. Der andere Einzeldraht 11, 12 mündet an der Umlenkstelle 23 tangential in die Abschnittskante 21, 22. Der andere Einzeldraht 11, 12 wird also beim Übergang in die Abschnittskante 21, 22 mit einem größeren Radius gekrümmt als der scharf umgelenkte Einzeldraht 11, 12.

Das Geflecht 10 weist dabei, wie in Fig. 1c zu erkennen, in der direkt an die Abschlusskante 20 angrenzenden Zellreihe 29 etwas größere Zellen bzw, Zellformen auf, die sich von den weiter entfernten Zellen im Geflecht unterscheiden. Dies liegt daran, dass der zusätzliche zweite Einzeldraht 12, der am selben Umlenkstift 23a bzw. derselben Umlenkstelle 23 wie der erste Einzeldraht 11 umgelenkt wird, zum Umlenkstift 23a hingebogen ist. Die lokale Änderung des Flechtwinkels des zweiten Einzeldrahtes 12 durch die gemeinsame Umlenkung an einer Umlenkstelle 23 der Abschlusskante 20 ist auf ein oder zwei Zellenabstände von der Abschlusskante 20 beschränkt.

Auch in diesem Fall setzen sich die Maschen 15 des Gittergeflechts 10 gleichmäßig in die Profilabschnitte 25 fort, wobei im Gittergeflecht 10 außerhalb der Profilabschnitte 25 zwei verschiedene Maschengrößen vorliegen, nämlich kleinere und größere Maschen. Die größeren Maschen entstehen durch die gemeinsame Umlenkung bzw. Führung mehrerer Einzeldrähte an einer einzigen Umlenkstelle 23. Die entsprechend größeren Maschen 15 setzen sich entlang der Abschlusskante 20 fort und grenzen direkt an diese an. Weiter im Inneren der Profilabschnitte 25 liegt die kleinere Maschengröße vor, die auch im Geflecht außerhalb der Profilabschnitte 25 ausgebildet ist.

Bei dem Ausführungsbeispiel gemäß Fig. 1c ist das erfindungsgemäße Prinzip, wonach an jeder Umlenkstelle 23 der jeweiligen Abschnittskante 21, 22 ein Einzeldraht 11, 12 in die Abschnittskante 21, 22 mündet, erfüllt. Grundsätzlich können mehrere Drähte, insbesondere mehrere Einzeldrähte 11, 11', 12, 12', an ein und derselben Umlenkstelle 23 in die Abschnittskante 21, 22 überführt werden. Wie zuvor erläutert, kann eine Umlenkung von mehreren Einzeldrähten 11, 11', 12, 12' an ein und derselben Umlenkstelle 23 aus herstellungsbedingten Gründen zweckmäßig sein, um eine hohe Feinmaschigkeit zu des Gittergeflechts 10 erreichen. Die Feinmaschigkeit des Gittergeflechts 10 wird durch den Durchmesser der Umlenkstifte begrenzt, um die die Einzeldrähte 11, 11', 12, 12' zur Herstellung des Gittergeflechts 10 geführt sind. Durch die Umlenkung mehrerer Drähte 11, 11', 12, 12' an ein und demselben Umlenkstift kann die Feinmaschigkeit erhöht werden. Dabei werden an den Stellen, an denen die Einzeldrähte 11, 11', 12, 12' um die Umlenkstifte geführt werden, Umlenkstellen 23 gebildet.

Generell gilt, dass mehrere Drähte 11, 11', 12, 12' an ein und derselben Umlenkstelle 23 in der Abschnittskante 21, 22 zusammengefasst sein können, wodurch sich die Maschengröße der an die Abschnittkante 21, 22 angrenzenden Maschen 15 gegenüber den Maschen 15 innerhalb des Gittergeflechts 10 vergrößert. Die Zusammenfassung der Drähte bzw. Einzeldrähte 11, 11', 12, 12', also die Bildung eines Drahtstrangs, erfolgt nur in der jeweiligen Abschnittskante 21, 22. Innerhalb des Drahtstrangs können die Einzeldrähte 11, 11', 12, 12' parallel bzw. litzenartig aneinander angrenzend verlaufen oder beispielsweise durch Verdrillen miteinander verbunden sein. Bei der Zusammenfassung von mehreren Drähten 11, 11', 12, 12' an ein und derselben Umlenkstelle 23 ist ein Draht 11, 12 abrupt in die Abschnittskante 21, 22 umgelenkt. Ein anderer Draht 11', 12', der vor der Umlenkstelle 23 um eine Maschenbreite von dem abrupt umgelenkten Draht 11, 12 beabstandet bzw. versetzt ist, mündet tangential in die Abschnittskante 21, 22.

Das Ausführungsbeispiel gemäß Fig. 1d, 1e ist ähnlich aufgebaut, wie das Ausführungsbeispiel gemäß Fig. 1c, wobei im Unterschied zum Ausführungsbeispiel gemäß Fig. 1c im Bereich einer Abschnittskante 21, 22 nur eine einzige Umlenkstelle 23 bzw. ein einziger Umlenkstift 23a angeordnet ist, an dem ein aus dem Geflecht kommender Einzeldraht umgelenkt wird. Der im Bereich der Spitze 26 angeordnete Umlenkstift 23a dient nicht der Umlenkung eines aus dem Geflecht kommenden Einzeldrahtes, sondern der Bildung der Spitze 26. Diese gilt auch für die anderen Ausführungsbeispiele.

Wie im Zusammenhang mit Fig. 1c beschrieben, wird an der Umlenkstelle 23 ein erster bzw. zweiter Draht 11, 12 unter Änderung der Spiralrichtung umgelenkt. Ein weiterer erster bzw. zweiter Draht 11', 12' mündet tangential an dem jeweiligen Umlenkstift 23a bzw. der Umlenkstelle 23 in die Abschlusskante 20 bzw. die jeweilige Abschnittskante 21, 22.

An dieser Stelle wird darauf hingewiesen, dass die Umlenkstifte 23a nur zum besseren Verständnis dargestellt sind und zur Flechteinrichtung gehören. Die Vorrichtung an sich umfasst keine Umlenkstifte 23a. Der Begriff "Umlenkstift" wird im Rahmen dieser Anmeldung alternativ zu dem Begriff "Umlenkstelle" gebraucht und ist insofern austauschbar.

Wie insbesondere in Fig. 1e zu erkennen, ist die Umlenkstelle 23 vom Kreuzungsendpunkt 17 beabstandet, insbesondere um wenigstens eine Maschenbreite, konkret um zwei Maschenbreiten. Das bedeutet, dass der umzulenkende Einzeldraht im Bereich der bereits ausgebildeten Abschnittskante 21, 22 in die Abschlusskante 20 mündet bzw. übergeht. Dies gilt entsprechend für die erste in Richtung der Spitze 26 gesehene Umlenkstelle 23 des Geflechts gemäß Figur 1c.

Die Abschnittskante 21, 22 bei den Geflechten gemäß Fig. 1c, 1e vor der einzigen bzw. ersten Umlenkstelle 23 ist aus mindestens zwei, insbesondere konkret aus zwei Drähten gebildet, die vor der Umlenkstelle 23 tangential zusammengeführt sind und dann nebeneinander verlaufen. Diese Konstruktion führt zu einer verbesserten Stabilität der Abschlusskante 20. Außerdem vermindert sich das Risiko der Verzerrung des Geflechts.

Es ist möglich, dass die beiden Drähten 11, 11' bzw. die beiden Drähte 12, 12', die die Abschnittskanten 21 bzw. 22 vor der ersten bzw. einzigen Umlenkstelle 23 bilden nicht exakt am Kreuzungsendpunkt 17 zusammenlaufen, sondern in Richtung der Spitze 26 gesehen näher an der Umlenkstelle 23. Im Bereich der Endüberkreuzung 17 verlaufen die Drähte 11, 11' bzw. 12, 12' in etwa tangential. Im Bereich der ersten bzw. einzigen Umlenkstelle 23 (Figuren 1c, 1e) verlaufen die Drähte der Abschlusskante nebeneinander. Dadurch besteht eine definierte Abschlusskante 20 bereits an der Stelle, an der die Umlenkung der weiteren Drähte 12, 12' bzw. 11, 11' erfolgt.

Die parallel nebeneinander verlaufenden Drähte 11, 11' und 12, 12' sind im Bereich vor der ersten bzw. einzigen Umlenkstelle 23 nicht miteinander verbunden, sondern verlaufen frei nebeneinander. Alternativ können die Drähte 11, 11' bzw. 12, 12' miteinander verbunden sein, beispielsweise verdrillt, verflochten oder anderweitig miteinander verbunden sein. Dasselbe gilt für die im Verlauf der Abschlusskante 20 zugeführten Drähte 11, 11' bzw. 12, 12', die ebenfalls entweder unverbunden nebeneinander oder miteinander verbunden sein können.

Anhand des Ausführungsbeispiels gemäß Fig. 1f wird verdeutlicht, dass eine unterschiedliche Anzahl von Umlenkstellen 23 pro Abschnittskante 21, 22 möglich ist, beispielsweise zwei Umlenkstellen. Der Grundaufbau entspricht der Struktur gemäß Fig. 1a.

In Fig. 2 ist ein weiteres Ausführungsbeispiel der erfindungsgemäßen Vorrichtung dargestellt, wobei das Gittergeflecht 10 eine Abschlusskante 20 umfasst, die mehrere Profilabschnitte 25 mit jeweils einer abgerundeten glatten Abschnittskante 21 aufweist. Der Aufbau der Abschnittskante folgt im Wesentlichen gemäß dem Ausführungsbeispiel nach Fig. 1, wobei die Drahtanzahl der die Abschnittskante 21 bildenden Drähte 11, 12 zu einem Scheitelpunkt 24 des Profilabschnitts 25 hin ansteigt. Auch bei dem Ausführungsbeispiel gemäß Fig. 2 werden der Abschnittskante 21 mehrere Drähte 11, 12 zugeführt, die umgelenkt werden, um dem Verlauf der Abschnittkante 21 zu folgen. Die Umlenkung der Drähte 11, 12 erfolgt an Umlenkstellen 23, die entlang der Abschnittskante 21 regelmäßig beabstandet angeordnet sind. Zwischen den Umlenkstellen 23 erhöht sich die Drahtanzahl, wie bei dem Ausführungsbeispiel gemäß Fig. 1, um jeweils einen Draht 11, 12.

Der Unterschied zwischen dem Ausführungsbeispiel gemäß Fig. 2 und dem Ausführungsbeispiel gemäß Fig. 1 besteht darin, dass die Drähte 11, 12 die die Profilabschnitte 25 bilden, im Scheitelpunkt 24 des jeweiligen Profilabschnitts 25 einstückig miteinander verbunden sind. Mit anderen Worten sind die Profilabschnitte 25 jeweils durch Drähte 11, 12 gebildet, die im Bereich der Abschlusskante 22 Schlaufen bilden. Dabei bildet der Scheitelpunkt 24 eine fiktive Unterteilung des jeweiligen Drahtelements 11, 12 in einen ersten Drahtabschnitt 11a, 12a und einen zweiten Drahtabschnitt 11b, 12b. Der erste Drahtabschnitt 11a, 12a entspricht im Wesentlichen dem ersten Draht 11 gemäß Fig. 1. Der zweite Drahtabschnitt 11b, 12b entspricht im Wesentlichen dem zweiten Draht 12 gemäß Fig. 1. Das bedeutet, dass die ersten Drahtabschnitte 11a, 12a in der zeichnerischen Darstellung gemäß Fig. 2 von links unten nach rechts oben verlaufen und die zweiten Drahtabschnitte 11b, 12b von links oben nach rechts unten.

Die Schlaufen 13, 14 sind jeweils durch die ersten und zweiten Drahtabschnitte 11a, 12a, 11b, 12b begrenzt. Dabei kreuzen sich die ersten Drahtabschnitte 11a, 12a und die zweiten Drahtabschnitte 11b, 12b in unmittelbarer Nähe zur Abschlusskante 20. Die Form der Schlaufen 13, 14 wird im Folgenden anhand des Verlaufs eines ersten Drahts 11 näher erläutert und gilt analog für die Schlaufenbildung eines zweiten Drahts 12 bzw. weiteren Drahts:

Der erste Draht 11 weist einen ersten Drahtabschnitt 11a auf, der in der schematischen Darstellung gemäß Fig. 2 von links unten nach rechts oben verläuft. Der erste Drahtabschnitt 11a trifft im Verlauf auf eine Umlenkstelle 23 der Abschnittskante 21. An der Umlenkstelle 23 wird der erste Drahtabschnitt 11a umgelenkt und ändert somit seine Spiralrichtung. Die Umlenkung des ersten Drahtabschnitts 11a erfolgt derart, dass der erste Drahtabschnitt 11a im Bereich der Abschnittskante 21 in Richtung zum Scheitelpunkt 24 geführt ist. Der erste Drahtabschnitt 11a folgt im Verlauf der Abschnittskante 21 der Krümmung der Abschnittskante bis zum Scheitelpunkt 24. Ausgehend vom Scheitelpunkt 24 beginnt der zweite Drahtabschnitt 11b des ersten Drahts 11. Der zweite Drahtabschnitt 11b folgt der Krümmung der Abschnittskante 21 weiter bis zu einer Umlenkstelle 23. An der Umlenkstelle 23 wird der zweite Drahtabschnitt 11b umgelenkt und in das Gittergeflecht 10 zurückgeführt. Dabei weist der zweite Drahtabschnitt 11b eine zum ersten Drahtabschnitt 11a gegenläufige Spiralrichtung auf. Daher kreuzt der zweite Drahtabschnitt 11b im weiteren Verlauf den ersten Drahtabschnitt 11a. Der zweite Drahtabschnitt 11b verläuft im Geflecht 10 gemäß der zeichnerischen Darstellung nach Fig. 2 von links oben nach rechts unten. Ausgehend vom Kreuzungspunkt des ersten Drahtabschnitts 11a mit dem zweiten Drahtabschnitt 11b über die Umlenkstellen 23 und den Scheitelpunkt 24 wird die erste Schlaufe 13 des ersten Drahts 11 gebildet. Der zweite Draht 12 weist einen analogen Verlauf auf und bildet eine zweite Schlaufe 14. Die zweite Schlaufe 14 ist größer als die erste Schlaufe 13.

Die erste Schlaufe 13 und die zweite Schlaufe 14 sind im Bereich der Abschlusskante 20 derart überlagert, dass sich die ersten und zweiten Drahtabschnitte 11a, 12a, 11b, 12b des ersten und zweiten Drahts 11, 12 entlang der Abschnittskante 21 überlappen. Zumindest im Scheitelpunkt 24 sind der erste und der zweite Draht 11, 12 miteinander verbunden. Beispielsweise können der erste und der zweite Draht 11, 12 im Bereich der Abschnittskante 21, insbesondere zwischen den Umlenkstellen 23 des ersten Drahts 11, miteinander verbunden, insbesondere verdrillt sein.

Für das Ausführungsbeispiel gemäß Fig. 2 gilt, dass die Abschnittskanten 21, 22 nicht zu einer scharfen Spitze konvergieren, sondern gehen kontinuierlich ineinander über. Die Abschlusskante 20 weist also eine gleichmäßig gekrümmte, insbesondere wellenförmige, Kontur auf. Insbesondere können die Abschnittskanten 21, 22 an der abgeflachten Spitze 26 des Profilabschnitts 25 Tangenten aufweisen, die sich unter einem flachen Winkel (wenigstens 90°) schneiden.

Für alle Ausführungsbeispiele gilt, dass die Abschnittskanten 21, 22 zumindest in einer Abwicklung des Gittergeflechts 10 geradlinig ausgebildet sein können. Die beiden Abschnittskanten 21, 22 sind zumindest in einer Abwicklung des Gittergeflechts 10 nicht konkav, sondern insbesondere geradlinig oder konvex ausgebildet. Mit anderen Worten begrenzen die Abschnittskanten 21, 22 den jeweiligen Profilabschnitt 25 entweder geradlinig oder sind nach außen gewölbt.

Ferner gilt allgemein, dass sich die ersten und zweiten Drähte 11, 12 innerhalb des Gittergeflechts 10 mehrfach überkreuzen und Maschen 15 bilden. Die Maschen 15 sind vorzugsweise durch jeweils zwei erste und zwei zweite Drähte 11, 12 begrenzt. Vorzugsweise sind die Maschen 15 rautenförmig ausgebildet.

Folgende Merkmale bevorzugter Ausführungsbeispiele werden allgemein offenbart:

Der Flechtwinkel der Drähte 11, 12 kann im Bereich des Geflechtes 10, d.h. außerhalb der Abschlusskante 20 im Wesentlichen derselbe sein. Eine lokale Änderung des Flechtwinkels durch die gemeinsame Umlenkung mehrerer, insbesondere von zwei Drähten an einer Umlenkstelle der Abschlusskante ist auf ein oder zwei Zehenabstände von der Abschlusskante 20 beschränkt.

Die Umlenkung kann mit einem Winkel von etwa 90° erfolgen. Der Betrag des Flechtwinkels eines Drahtes bzw. aller Drähte vor und nach der Umlenkung kann derselbe sein.

Im Bereich der Abschlusskante 20 können die zusammengeführten Drähte 10, 11 verdrillt sein.

Die Umlenkung der Drähte 11, 12 erfolgt nur auf Höhe der Abschlusskante 20. Es wird nur jeweils ein einziger Draht 11, 12 umgelenkt. Vor der Umlenkstelle, also von der Abschlusskante 20 beabstandet, erfolgt keine Zusammenfassung mehrerer Drähte, die gemeinsam zur Umlenkstelle geführt sind. Bei der Umlenkung mehrerer Einzeldrähte an derselben Umlenkstelle (Umlenkstift), treffen sich die Einzeldrähte an der Umlenkstelle. Vor der Umlenkstelle, also von der Abschlusskante 20 beabstandet, sind die Einzeldrähte getrennt geführt.

Allgemein wird eine bevorzugte Ausführung der medizinischen Vorrichtung zur Einfuhr in ein Hohlorgan mit einem Hohlkörper offenbart, die ein Geflecht aus Drahtelementen mit einer Reihe von Endmaschen aufweist, die ein axiales Geflechtende begrenzen. Die Endmaschen umfassen äußere Drahtelemente, die eine profilierte Abschlusskante mit mehreren Abschnittskanten des Geflechts bilden und in innere Drahtelemente übergehen, die innerhalb des Geflechts angeordnet sind.

Ein erster Abschnitt der Abschlusskante und ein zweiter Abschnitt der Abschlusskante weisen jeweils mehrere äußere Drahtelemente auf, die zusammen einen umlaufenden Rand der Abschlusskante bilden. Der umlaufende Rand ist kronenartig, insbesondere wellenartig oder zackenartig, profiliert. Der Rand weist mehrere Spitzen und Täler auf, wobei jeweils ein erster und zweiter Abschnitt der Abschlusskante zwei Abschnittskanten mit unterschiedlichen Spiralrichtungen entsprechen. Mehrere Abschnittskantenpaare sind auf dem Umfang der Abschlusskante verteilt angeordnet.

Die äußeren Drahtelemente des ersten Abschnitts sind zur Bildung der Abschlusskante entlang dieser einander unmittelbar nachgeordnet. Die äußeren Drahtelemente weisen jeweils eine erste Axialkomponente auf, die in Längsrichtung L des Hohlkörpers verläuft.

Die äußeren Drahtelemente des zweiten Abschnitts sind zur Bildung der Abschlusskante entlang dieser einander unmittelbar nachgeordnet. Die äußeren Drahtelemente weisen jeweils eine zweite Axialkomponente auf, die in Längsrichtung L des Hohlkörpers verläuft. Die zweite Axialkomponente ist der ersten Axialkomponente entgegengesetzt, wobei die beiden Axialkomponenten auf dieselbe Umlaufrichtung des Randes bezogen sind.

Der Hohlkörper hat allgemein eine längliche Form mit einer Längsrichtung und ist dazu geeignet, durch ein Zufuhrsystem, beispielsweise durch einen Katheter in ein Hohlorgan, insbesondere in ein menschliches Hohlorgan eingeführt zu werden. Dabei entspricht die Längsrichtung des Hohlkörpers derjenigen Richtung, in der der Hohlkörper durch das Zufuhrsystem bewegt und im Hohlorgan freigesetzt wird. Der Begriff Längsrichtung des Hohlkörpers entspricht somit dem Begriff Bewegungsrichtung des Hohlkörpers im Zufuhrsystem. Die Bewegungsrichtung des Hohlkörpers im Zufuhrsystem kann die Vorschubbewegung umfassen beim Freisetzen des Hohlkörpers ebenso wie die Rückzugsbewegung beim Wiedereinziehen des Hohlkörpers in das Zufuhrsystem.

Unter dem Begriff Endmaschen werden die äußeren Maschen des Geflechts verstanden, die ein Geflechtende begrenzen. Bei einem länglichen Hohlkörper handelt es sich bei dem Geflechtende um ein axiales Geflechtende. Die äußeren Drahtelemente der Endmaschen sind die auf der Außenseite der Endmaschen angeordneten Drahtelemente, die die Begrenzung des Geflechtendes darstellen. Die äußeren Drahtelemente bilden dabei eine Abschlusskante des Geflechts und gehen in innere Drahtelemente über, die innerhalb des Geflechts angeordnet sind.

Die äußeren Drahtelemente können somit auch als kantenbildende Drahtelemente und die inneren Drahtelemente als geflechtbildende Drahtelemente bezeichnet werden.

Der erste Abschnitt der Abschlusskante und der zweite Abschnitt der Abschlusskante bilden jeweils eine Teilkante, die das Geflecht in Längsrichtung des Hohlkörpers begrenzt. Jeder Abschnitt weist mehrere äußere Drahtelemente auf bzw. ist aus mehreren äußeren Drahtelementen gebildet, die zusammen einen umlaufenden Rand der Abschlusskante bilden. Der umlaufende Rand ist derart angepasst, dass das axiale Geflechtende des Hohlkörpers in ein Zufuhrsystem einziehbar ist. Dies bedeutet, dass das Hohlorgan in Längsrichtung mit dem umlaufenden Rand in das Zufuhrsystem rückholbar ist, nachdem der Hohlkörper vollständig oder zumindest überwiegend aus dem Zufuhrsystem entlassen wurde.

Die äußeren Drahtelemente der beiden Abschnitte sind zur Bildung der Abschlusskante entlang dieser jeweils einander unmittelbar nachgeordnet. Dies bedeutet, dass die äußeren Drahtelemente des ersten Abschnitts eine erste Einheit des umlaufenden Randes bilden, die sich dadurch auszeichnet, dass die äußeren Drahtelemente dieser ersten Einheit jeweils eine erste Axialkomponente aufweisen, die in Längsrichtung des Hohlkörpers verläuft.

Dasselbe gilt in analoger Weise für die äußeren Drahtelemente des zweiten Abschnitts, die zur Bildung der Abschlusskante entlang dieser einander unmittelbar nachgeordnet sind und eine zweite Einheit des umlaufenden Randes bilden. Die äußeren Drahtelemente des zweiten Abschnitts zeichnen sich dadurch aus, dass diese jeweils eine zweite Axialkomponente aufweisen, die in Längsrichtung des Hohlkörpers verläuft. Die beiden Axialkomponenten sind in entgegengesetzter Richtung angeordnet, wobei die beiden Axialkomponenten auf dieselbe Umlaufrichtung des Randes bezogen sind. Dies bedeutet, dass eine Axialkomponente in Längsrichtung des Hohlkörpers nach vorne und die andere Axialkomponente in Längsrichtung des Hohlkörpers nach hinten weist. Mit anderen Worten weist eine der beiden Axialkomponenten in proximale Richtung, d.h. in Richtung des Anwenders und die andere der beiden Axialkomponenten in distale Richtung, d.h. vom Anwender bzw. Arzt weg. Die unterschiedliche Richtung der Axialkomponenten wird dadurch ausgedrückt, dass diese auf dieselbe Umlaufrichtung des Randes bezogen sind. Unter derselben Umlaufrichtung des Randes wird beispielsweise die Bewegung eines imaginären Punktes auf dem Rand in ein und derselben Richtung verstanden. Dabei kommt es nicht darauf an, ob die Bewegung dieses imaginären Punktes im Uhrzeigersinn oder im Gegenuhrzeigersinn auf dem Rand verläuft. Für die Definition der Richtung der Axialkomponenten genügt es, wenn die Umlaufrichtung bzw. der imaginäre Umlauf im gleichen Sinn erfolgt.

Durch die neue Flechtart ist das Geflecht so aufgebaut, dass die Endkontur des Geflechts, d.h. die Kontur im Bereich der Abschlusskante zumindest an einem axialen Geflechtende keine überstehenden Kanten, bzw. keine entgegen der Einzugsrichtung des Geflechtendes in das Zufuhrsystem vorstehenden Kanten aufweist. Dies gilt für die zusammengehörenden Abschnittskanten der Abschlusskante, die insgesamt kronenartig, insbesondere wellenartig oder zackenartig, profiliert ist.

Generell kann das Gittergeflecht zwei axiale Enden aufweisen, die erfindungsgemäß ausgebildet sind, insbesondere beidseitig durch die erfindungsgemäßen Abschlusskanten begrenzt sind. Es ist auch möglich, dass nur ein axiales Ende eine erfindungsgemäße Abschlusskante und das andere axiale Ende eine schräge Umlaufkante aufweist, wie in DE 102009056450 offenbart.

Die im Zusammenhang mit den vorgenannten Ausführungsbeispielen beschriebenen Merkmale können einzeln miteinander kombiniert werden.

### Bezugszeichenliste

- 10: Gittergeflecht
- 11: erster Draht
- 11a, 12a: erster Drahtabschnitt
- 11b, 12b: zweiter Drahtabschnitt
- 12: zweiter Draht
- 13: erste Schlaufe
- 14: zweite Schlaufe
- 15: Masche
- 16: kantenbildender Draht
- 17: Kreuzungsendpunkt
- 20: Abschlusskante
- 21, 22: Abschnittskante
- 23: Umlenkstelle
- 24: Scheitelpunkt
- 25: Profilabschnitt
- 26: Spitze
- 27: Fortsatz

## Patentansprüche

1. Medizinische Vorrichtung zur Einfuhr in ein Hohlorgan mit einem komprimierbaren und expandierbaren Gittergeflecht (10), das eine Vielzahl erster Drähte (11) und eine Vielzahl zweiter Drähte (12) umfasst, die jeweils spiralförmig um eine gemeinsame Längsachse gewunden sind und unterschiedliche Spiralrichtungen aufweisen derart, dass sich die ersten Drähte (11) und die zweiten Drähte (12) zur Bildung von Maschen (15) kreuzen, wobei das Gittergeflecht (10) eine profilierte, umlaufende Abschlusskante (20) aufweist, die mehrere Profilabschnitte (25) mit jeweils glatten Abschnittskanten (21, 22) umfasst, die sich in unterschiedliche Spiralrichtungen entlang des Profilabschnitts (25) erstrecken und zu einer Spitze (26) des Profilabschnitts (25) konvergieren, wobei die beiden zur Spitze (26) konvergierenden Abschnittskanten (21, 22) jeweils durch wenigstens zwei Drähte (11, 12) gebildet sind und jeweils eine, der Spitze (26) entlang der Abschnittskante (21, 22) unmittelbar nachgeordnete, erste Umlenkstelle (23) aufweisen, an der ein Draht (11, 12) umgelenkt und der Abschnittskante (21, 22) zugeführt ist derart, dass sich der umgelenkte Draht (11, 12) entlang der Abschnittskante (21, 22) in eine andere Spiralrichtung als innerhalb des Gittergeflechts (10) erstreckt, und wobei sich die Anzahl der Drähte (11, 12) jeder Abschnittskante (21, 22) jeweils an der Umlenkstelle (23) ändert, insbesondere sich in Richtung zur Spitze (26) des Profilabschnitts (26) erhöht,
**dadurch gekennzeichnet, dass**
der umgelenkte Draht (11, 12) ein Einzeldraht ist, der nur an der jeweiligen Abschnittskante (21, 22) der Abschlusskante (20) umgelenkt ist.

2. Medizinische Vorrichtung nach Anspruch 1,
**dadurch gekennzeichnet, dass**
der Flechtwinkel des Einzeldrahtes vor der Umlenkstelle (23) dem Flechtwinkel des Einzeldrahtes nach der Umlenkstelle (23) entspricht.

3. Medizinische Vorrichtung nach Anspruch 1 oder 2,
**dadurch gekennzeichnet, dass**
sich die Abschnittskante (21, 22) auf beiden Seiten der Umlenkstelle (23) erstreckt, wobei die Abschnittskante (21, 22) vor dem umgelenkten Einzeldraht aus mindestens zwei Drähten (11, 11', 12, 12') gebildet ist.

4. Medizinische Vorrichtung nach Anspruch 3,
**dadurch gekennzeichnet, dass**
die beiden Drähte (11, 11', 12, 12') der Abschnittskante (21, 22) vor dem ersten umgelenkten Einzeldraht tangential zusammengeführt sind.

5. Medizinische Vorrichtung nach einem der vorhergehenden Ansprüche,
**dadurch gekennzeichnet, dass**
die in der Spitze (26) zusammenlaufenden Drähte (11, 12) miteinander verdrillt sind und einen Fortsatz (27) bilden, der sich im Wesentlichen parallel zur Längsachse des Gittergeflechts (10) erstreckt.

6. Medizinische Vorrichtung nach Anspruch 5,
**dadurch gekennzeichnet, dass**
der Fortsatz (27) wenigstens ein Verbindungselement, insbesondere eine Hülse, und/oder ein Markerelement, insbesondere einen Röntgenmarker, aufweist.

7. Medizinische Vorrichtung nach einem der vorhergehenden Ansprüche,
**dadurch gekennzeichnet, dass**
beide Drähte (11, 12), insbesondere alle Drähte (11, 12), die gemeinsam eine Abschnittskante (21, 22) bilden, beim Übergang vom Gittergeflecht (10) in die Abschlusskante (20) an jeweils einer Umlenkstelle (23) umgelenkt sind.

8. Medizinische Vorrichtung nach einem der vorhergehenden Ansprüche,
**dadurch gekennzeichnet, dass**
die Abschnittskanten (21, 22) jeweils einen Draht (11, 12) aufweisen, der in der Abschnittskante (21, 22) dieselbe Spiralrichtung wie im Gittergeflecht (10) aufweist.

9. Medizinische Vorrichtung nach einem der vorhergehenden Ansprüche,
**dadurch gekennzeichnet, dass**
die Drähte (11, 12) entlang der Abschnittskante (21, 22), insbesondere an den Übergangsstellen (23), miteinander verbunden sind.

10. Medizinische Vorrichtung nach einem der vorhergehenden Ansprüche,
**dadurch gekennzeichnet, dass**
die Abschnittskanten (21, 22) jeweils mehrere Umlenkstellen (23) aufweisen.

11. Medizinische Vorrichtung, insbesondere zum Entfernen von Konkrementen aus Körperhohlorganen, mit einem komprimierbaren und expandierbaren Gittergeflecht (10), das eine profilierte, umlaufende Abschlusskante (20) aufweist, die mehrere Profilabschnitte (25) mit jeweils einer glatten, abgerundeten Abschnittskante (21) umfasst, wobei die Abschnittskante (21) aus wenigstens zwei Drähten (11, 12) gebildet ist, die sich entlang der Abschnittskante (21) überlappende Schlaufen (13, 14) bilden und in einem Scheitelpunkt (24) der Abschnittskante (21) verbunden sind, der die Drähte (11, 12) in jeweils einen ersten Drahtabschnitt (11a, 12a) und einen zweiten Drahtabschnitt (11b, 12b) unterteilt, wobei der erste Drahtabschnitt (11a, 12a) und der zweite Drahtabschnitt (11b, 12b) in jeweils unterschiedlichen Spiralrichtungen spiralförmig um eine gemeinsame Längsachse gewunden sind derart, dass sich der erste Drahtabschnitt (11a, 12a) und der zweite Drahtabschnitt (11b, 12b) zur Bildung von Maschen (15) im Gittergeflecht (10) kreuzen.

## Claims

1. A medical device for introduction into a hollow organ with a compressible and expandable latticework mesh (10) comprising a plurality of first wires (11) and a plurality of second wires (12), which are each spirally wound around a common longitudinal axis and have different spiral directions in such a way that the first wires (11) and the second wires (12) cross each other to form meshes (15), wherein the latticework mesh (10) has a profiled, circumferential concluding edge (20) which has several profile sections (25) each with smooth section edges (21, 22) which extend in different spiral directions along the profile section (25) and converge to a tip (26) of the profile section (25), wherein the two section edges (21, 22) converging to the tip (26) are each formed by at least two wires (11, 12) and each have one first deflection point (23), immediately downstream of the tip (26) along the section edge (21, 22), at which a wire (11, 12) is deflected and taken to the section edge (21, 22) in such a way that the deflected wire (11, 12) extends along the section edge (21, 22) in a spiral direction different from within the latticework mesh (10), and wherein the number of wires (11, 12) of each section edge (21, 22) changes at the respective deflection point (23), in particular increases in the direction of the tip (26) of the profile section (25),
**characterised in that**
the deflected wire (11, 12) is a single wire which is only deflected on the respective section edge (21, 22) of the concluding edge (20).

2. The medical device according to claim 1,
**characterised in that**
the braid angle of the single wire before the deflection point (23) corresponds with the braid angle of the single wire after the deflection point (23).

3. The medical device according to claim 1 or 2,
**characterised in that**
the section edge (21, 22) extends on both sides of the deflection point (23), wherein the section edge (21, 23) before the deflected single wire is formed of at least two wires (11, 11', 12, 12').

4. The medical device according to claim 3,
**characterised in that**
the two wires (11, 11', 12, 12') of the section edge (21, 22) are tangentially brought together before the first deflected single wire.

5. The medical device according to any one of the preceding claims,
**characterised in that**
the wires (11, 12) converging at the tip (26) are twisted together and form a projection (27) which essentially extends in parallel to the longitudinal axis of the latticework mesh (10).

6. The medical device according to claim 5,
**characterised in that**
the projection (27) comprises at least one connecting element, in particular a sleeve and/or a marker element, in particular an X-ray marker.

7. The medical device according to any one of the preceding claims,
**characterised in that**
the two wires (11, 12), in particular all wires (11, 12), which form a common section edge (21, 22), at the transition from the latticework mesh (10) to the concluding edge (20) are each deflected at one deflection point (23).

8. The medical device according to any one of the preceding claims,
**characterised in that**
the section edges (21, 22) each comprise one wire (11, 12) which in the section edge (21, 22) has the same spiral direction as in the latticework mesh (10).

9. The medical device according to any one of the preceding claims,
**characterised in that**
the wires (11, 12) along the section edge (21, 22), particularly at the transition points (23) are connected to each other.

10. The medical device according to any one of the preceding claims,
**characterised in that**
the section edges (21, 22) each comprise several deflection points (23).

11. The medical device, in particular for removing concretions from hollow organs of the body, with a compressible and expandable latticework mesh (10) which comprises a profiled, circumferential concluding edge (20) which has several profile sections (25) each with a smooth, rounded section edge (21), wherein the section edge (21) is formed of at least two wires (11, 12) which along the section edge (21) form overlapping loops (13, 14) and are connected at an apex (24) of the section edge (21), which divides the wires (11, 12) into a first wire section (11a, 12a) and second wires section (11b, 12b), wherein the first wire section (11a, 12a) and the second wire section (11b, 12b) are wound around a common longitudinal axis in a spiral manner in different spiral directions in such a way that the first wire section (11a, 12b) and the second wire section (11b, 12b) cross each other to form meshes (15) in the latticework mesh (16).

## Revendications

1. Dispositif médical destiné à être introduit dans un organe creux comprenant un treillis (10) pouvant être comprimé et extensible qui comprend une pluralité de premiers fils (11) et une pluralité de seconds fils (12) qui sont enroulés respectivement en spirale sur un axe longitudinal commun et présentent différentes orientations de spirale de telle sorte que les premiers fils (11) et les seconds fils (12) se croisent pour former des mailles (15), dans lequel le treillis (10) présente un bord de clôture (20) circulaire profilé qui comprend plusieurs tronçons profilés (25) avec respectivement des bords de tronçons (21, 22) lisses qui s'étendent dans différentes orientations de spirale le long du tronçon profilé (25) et convergent vers une pointe (26) du tronçon profilé (25), dans lequel les deux bords de tronçons (21, 22) convergeant vers la pointe (26) sont formés respectivement par au moins deux fils (11, 12) et présentent respectivement un premier point de déviation (23) immédiatement après la pointe (26) le long du bord de tronçon (21, 22), sur lequel un fil (11, 12) est dévié et amené vers les bords de tronçons (21, 22) de telle sorte que le fil (11, 12) dévié s'étend le long du bord de tronçon (21, 22) dans une autre orientation de spirale qu'à l'intérieur du treillis (10), et dans lequel le nombre de fils (11, 12) de chaque bord de tronçon (21, 22) change respectivement sur le point de déviation (23), en particulier, augmente en direction de la pointe (26) du tronçon profilé (25),
**caractérisé en ce que**
le fil (11, 12) dévié est un fil individuel qui est dévié seulement sur le bord de tronçon (21, 22) respectif du bord de clôture (20).

2. Dispositif médical selon la revendication 1,
**caractérisé en ce que**
l'angle de tressage du fil individuel devant le point de déviation (23) correspond à l'angle de tressage du fil individuel derrière le point de déviation (23).

3. Dispositif médical selon la revendication 1 ou 2,
**caractérisé en ce que**
le bord de tronçon (21, 22) s'étend sur les deux côtés du point de déviation (23), dans lequel le bord de tronçon (21, 22) est formé d'au moins deux fils (11, 11', 12, 12') devant le fil individuel dévié.

4. Dispositif médical selon la revendication 3,
**caractérisé en ce que**
les deux fils (11, 11', 12, 12') du bord de tronçon (21, 22) sont dirigés conjointement en tangente devant le premier fil individuel dévié.

5. Dispositif médical selon l'une des revendications précédentes,
**caractérisé en ce que**
les fils (11, 12) passant conjointement dans la pointe (26) sont torsadés entre eux et forment un prolongement (27) qui s'étend essentiellement parallèlement à l'axe longitudinal du treillis (10).

6. Dispositif médical selon la revendication 5,
**caractérisé en ce que**
le prolongement (27) présente au moins un élément de liaison, en particulier une douille et/ou un élément marqueur, en particulier un marqueur radiographique.

7. Dispositif médical selon l'une des revendications précédentes,
**caractérisé en ce que**
les deux fils (11, 12), en particulier tous les fils (11, 12) qui forment conjointement un bord de tronçon (21, 22) sont déviés sur respectivement un point de déviation (23) lorsqu'ils passent du treillis (10) au bord de clôture (20).

8. Dispositif médical selon l'une des revendications précédentes,
**caractérisé en ce que**
les bords de tronçons (21, 22) présentent respectivement un fil (11, 12) qui présente dans le bord de tronçon (21, 22), la même orientation de spirale que dans le treillis (10).

9. Dispositif médical selon l'une des revendications précédentes,
**caractérisé en ce que**
les fils (11, 12) sont reliés entre eux le long du bord de tronçon (21, 22), en particulier sur les points de passage (23).

10. Dispositif médical selon l'une des revendications précédentes,
**caractérisé en ce que**
les bords de tronçons (21, 22) présentent respectivement plusieurs points de déviation (23).

11. Dispositif médical, en particulier pour éliminer des calculs d'organes du corps creux, comprenant un treillis (10) pouvant être comprimé et extensible qui présente un bord de clôture (20) circulaire profilé qui comprend plusieurs tronçons profilés (25) avec respectivement un bord de tronçon (21) lisse arrondi, dans lequel le bord de tronçon (21) est formé d'au moins deux fils (11, 12) qui forment des passants (13, 14) se chevauchant le long du bord de tronçon (21) et sont reliés en un sommet (24) du bord de tronçon (21), qui divise les fils (11, 12) en respectivement un premier tronçon de fil (11a, 12a) et un second tronçon de fil (11b, 12b), dans lequel le premier tronçon de fil (11a, 12a) et le second tronçon de fil (11b, 12b) sont enroulés respectivement en spirale dans différentes orientations de spirale sur un axe longitudinal commun de telle sorte que le premier tronçon de fil (11a, 12a) et le second tronçon de fil (11b, 12b) se croisent pour former des mailles (15) dans le treillis (10).
